# EUROPEAN PATENT APPLICATION

(11) **EP 2 743 347 A2**
(43) Date of publication of application: **18.06.2014**
(21) Application number: 11763748.8
(22) Date of filing: 08.08.2011
(51) Int. Cl.: C12N 9/16, C07K 14/24

(54) **NOVEL PHYTASE, METHOD FOR OBTAINING SAME AND USE THEREOF**

(71) Applicant: Fertinagro Nutrientes, S.L., 44195 Teruel (ES)
(72) Inventor: ALIGUE ALEMANY, Rosa, E-44195 Teruel (ES); ATARES REAL, Sergio, E-44195 Teruel (ES); MARQUES MASCARELL, Ramon, E-44195 Teruel (ES); MARTIN PEREZ, Julia, E-44195 Teruel (ES); ROMERO LOPEZ, Joaquin, E-44195 Teruel (ES); SALAET MADORRAN, Ignacio, E-44195 Teruel (ES)
(74) Representative: González López-Menchero, Álvaro Luis
(86) International application number: PCT/ES2011/070587
(87) International publication number: WO 2011/141613

(57) **Abstract**

The invention relates to an isolated *Serratia odorifera* gene encoding a protein or polypeptide with phytase activity, to a method for obtaining it in a yeast host cell such as *Pichia pastoris* for example, and to the use of the enzyme as an active ingredient in the manufacture of additives for feeds intended for animal nutrition and/or for the release of phosphate from organic manner coming from soil.

## Description

The present invention relates to a novel phytase gene, to the phytase enzyme encoded by said gene, to a method for obtaining the enzyme in a yeast host cell and to the use of the enzyme as an active ingredient in the manufacture of additives for feeds intended for animal nutrition and/or for the release of phosphate from organic matter coming from soil.

More particularly, the invention relates to an isolated *Serratia odorifera* gene encoding a protein or polypeptide with phytase activity, to a method for obtaining it in a yeast host cell such as *Pichia pastoris* for example, and to the use of the enzyme as an active ingredient in the manufacture of additives for feeds intended for animal nutrition.

Elemental or pure phosphorus (P) is rare in nature, it being usual to find it forming part of molecules including the phosphate group (PO4³⁻) bound to a carbon-based organic group in animal and plant tissues.

Phosphorus is an essential nutrient for plants and animals. Due to the lack of inorganic phosphorus of soil (the only form in which it is absorbed by plants), farmers are obliged to make supplies of phosphorus, part of which will be fixed by the plants, being converted into organic phosphorus, a certain proportion remaining in the soil however. Animals also need phosphorus in inorganic form, which they obtain from the inorganic phosphorus supplied directly in the diet and from the degradation of the organic phosphorus present in the various ingredients making up said diet.

Phytic acid or phytate is the main storage form of phosphorus in cereals, legumes, oleaginous seeds, with values comprised between 1-5% of the weight. More than two thirds of the phosphorus contained in cereals and leguminous vegetables are in phytate form, in addition to phosphorus, other cations such as calcium, magnesium, iron, zinc, as well as proteins and amino acids, are also sequestered by phytates.

However, monogastric animals (pigs and birds) are incapable of absorbing the phosphorus in phytate form, since they lack the enzymes necessary for digesting it, known as phytases. This means that between 20% and 40% of the phosphorus of plant origin ingested by monogastric animals is not assimilated, being excreted through feces with the consequent ecological problems. This pollution by phosphorus is a problem which is becoming increasingly important in areas in which intensive monogastric animal husbandry is practiced.

Furthermore, although some microorganisms present in the intestinal flora of the small intestine have the capacity to hydrolyze phytic acid, due to the location thereof in the intestine phosphorus is absorbed in a very small amount. In addition, an excess of phytate acts as an anti-nutritional factor as it chelates metal ions necessary for the animal such as calcium, copper and zinc, decreasing their nutritional availability.

These facts make it common to enrich the food of these animals with inorganic phosphorus, which increases the livestock production costs to a great extent, in addition to supplying extra non-assimilated phosphorus to the soil through the fertilization with manure, which breaks the natural equilibrium of this element, causing the eutrophication of surface water and the pollution of aquifers.

A few years ago and after a number of investigations, a possible solution to these problems, or at least their minimization, was contributed to the state of the art by providing the first phytase, an enzyme which hydrolyzes phytate and releases organic phosphorus and which, added to the food, makes phosphorus more accessible.

In this respect, phytases are meso-inositol hexaphosphate phosphohydrolases and catalyze the cleavage of the phosphate groups of phytic acid (IP6) or of phytate, resulting in a free phosphate group and a lower inositol phosphate ester (IP5-IP1). Phytases can be divided into 2 groups: microbial or fungal phytases (E.C. 3.1.3.8) or 3-phytase, which start to hydrolyze the phosphate group located in C1 or C3 of the inositol ring or plant phytases (E.C. 3.1.3.26) or 6-phytase, which preferably start to hydrolyze the phosphate group located in C6 of the inositol ring.

As mentioned in the article by Carmen Frontela et *al.,* "Empleo de fitasas como ingrediente funcional en alimentos" (Use of phytases as a functional ingredient in foods), (Carmen Frontela, Gaspar Ros, Carmen Martinez, Department of Food Technology, Nutrition and Bromatology, School of Veterinary Science, University of Murcia, Espinardo Campus, Murcia, Spain, 2008) for the purpose of achieving a complete degradation of the phytic acid content during the processing of foods, and thus increase the mineral availability, there are a number of investigations pointing to the use of phytases of exogenous origin as a strategy.

Thus, it is very important to know the optimal conditions of activity of the phytase used, taking into account that these conditions depend on the type of cereal which is being worked with. Most phytases coming from cereals have an optimal pH of activity between 4.5-5.6, whereas those coming from leguminous plants have an optimal pH at values of 7 or above. Nevertheless, there is evidence which shows that the use of phytases coming from microorganisms as is the case of fungi of the *Aspergillus spp.* genus is the simplest and most effective method for achieving the complete removal of phytic acid, causing a significant increase in the absorption of iron, which could mean that the use of these enzymes of microbial origin during the processing of foods could in the near future be feasibly used in the food industry for the purpose of increasing the mineral availability of the foods produced.

The use of phytase of microbial origin is currently authorized for use in animal food, and is aimed at increasing the availability of phytic acid phosphorus by reducing the fecal excretion thereof into the environment. In the case of the enzyme preparation of 6-phytase produced by *Aspergillus oryzae* (DSM 14223), it was authorized for chickens for fattening, laying hens, turkeys for fattening, piglets, pigs for fattening and sows by the European Commission Regulation (EC) no. 255/2005, and new data have currently been submitted in support of an application for extending the authorization of the use of this enzyme preparation to salmonids.

Phytases are extremely weak, expressing their maximum activity at pHs varying between 5.0 and 7.5, therefore the low pH values present in the stomach (pH 2-3) limit their activity, with some activity at a pH less than 3.0, therefore there is a considerable breakage of the phytate molecule of the diet, in the crop of chickens and in the stomach of non-ruminants, before the gastric secretion reduces the pH of the ingestion to a level that it too low to interrupt the enzymatic activity (Simons et *al.,* 1992). The enzyme furthermore tends to be strongly inhibited by an excess of substrate (phytate) and product (inorganic phosphorus) and by high temperatures (Power and Khon, 1993).

Synthetic phytases, which hydrolyze phytic acid, like intrinsic phytases of vegetables, are phosphomonoesterases capable of hydrolyzing phytic acid, producing inorganic orthophosphates and a small proportion of phosphoric esters, pentaphosphate to monophosphate, as intermediate products, and finally free myoinositol (Nayini and Markakis, 1986; Lasztity and Lasztity, 1988; Harland and Morris, 1995). The IUPAC-IUB (1976) has acknowledged 3-phytase (EC 3.1.3.8), isolated in animals and microorganisms (Reddy et al., 1982; Lasztity and Lasztity, 1988).

Exogenous phytases have been found in microorganisms such as fungi (*Saccharomyces cerevisae, Aspergillus* sp species), yeasts and bacteria (*Bacillus subtilis,* Pseudomonas).The phytase obtained from *Aspergillus sp* follows a certain order for the hydrolysis of the phytate molecule, i.e., after the phosphate group is released from position 3, it continues in the following order, 4, 5, 6 and 2 (Venekamp et *al.,* 1995).

The enzymes have activity at two different pHs, 2.5 and 5.5, being 40% less effective at the first pH. This is very important due to the fact that the greater absorption of phosphorus occurs in the proximity of the small intestine, where the pH of 5.5 is optimal for the phytase to act by hydrolyzing the phosphates, it first being necessary for the enzyme to be activated in the acidic medium of the stomach (pH 2.5) (Power and Khon, 1993).

The phytase produced by *Aspergillus ficuumm* is a purified glycoprotein with an enzymatic activity varying with temperature and pH changes. The optimal temperature of the enzyme is of 60 to 70°C. However, temperatures of 68°C for 10 minutes cause losses of 60% of activity (Nasi, 1990). The enzymes obtained from *Aspergillus niger* are thermostable, resistant to the pelletizing process and their activity is at least 5000 FTU/g. (Nasi, 1990).

There is evidence that phytases can significantly increase the use of phytic phosphorus (Qian et *al.,* 1997; Yi et *al.,* 1996a,b; Carlos et al., 1998; Maenz and Classen, 1998; Pan et *al.,* 1998; Denbow et *al.,* 1998).

Thus, Denbow et *al.* (1998) establish that the incorporation of phytases (400, 800, or 1,200 U/kg) to a basic diet low in phosphorus linearly increased the weight gain, the toe ash content and the tibial shear strength of birds and furthermore improved the digestibility of phosphorus (Nelson et *al.,* 1971; Denbow et *al.*, 1995; Kornegay et *al.,* 1996; Yi et *al*., 1996). These experiments establish that close to 821 U/kg of phytase are required to replace 1 g of inorganic phosphate as defluorinated phosphate, in terms of weight gain and ash percentage.

Simons et *al*. (1990), by adding phytase (750 U/kg) in a corn-soybean diet for chickens, increased the availability of phosphorus and calcium to 62% and decreased the excretion of phosphorus through feces and urine (2.0 g/kg MS). In addition, Perney (1991), using chickens for fattening fed with a basic diet based on corn-soybean, with 0.38% available phosphorus, with the addition of 500 U/kg phytase, obtained improvements in the tibial shear strength, without significant changes in the consumption and weight gain of the animals. These results were similar to those obtained by Edwards (1993), who observed little benefit due to the low levels of addition of phytase to phosphorus-deficient diets.

United States patent application US 2010/0092613, "Cloning and expression of a novel phytase" relates to a phytase obtained from a microorganism of the *Yersinia* genus with a molecular weight of 45.5 kDa, an optimal pH of 4-5, an optimal temperature of 50-60°C, an isoelectric point of 7.7 and a specific activity of 3,000 U/mg.

ES 2255281, "Sobreexpresión de genes de fitasa en sistemas de levadura" (Overexpression of phytase genes in yeast systems) relates to a method for producing phytase in yeast which comprises providing an appA gene isolated from bacterial cells, said appA gene encoding a protein or polypeptide with phytase activity, expressing said appA gene in a yeast strain and isolating the protein or polypeptide expressed, in which said protein or polypeptide has an increased thermostability in comparison with that of said protein or polypeptide expressed in a non-yeast host cell, as well as to a protein or polypeptide obtainable by means of said method and having phytase activity, with an optimal activity in the temperature range of 57-65°C at a pH of 2.5 to 3.5 or of 5.5.

Due to the foregoing, it would be desirable to provide a novel phytase the use of which does not entail the drawbacks of the phytases known in the state of the art, for example those related to:
- The pH difference between the stomach (pH 3.5) and the intestinal tract (pH 7.0), which makes it impossible for the enzyme to have a good activity with such a broad pH range;
- Thermostability also has an important role, since compound feeds in the granulation (pellet formation) phase undergo a heat shock of the order of 70°C which denatures the enzyme;
- The digestive tract proteases catabolize a large part of the enzyme;
and which, therefore, allows achieving more efficient results upon remaining active throughout the entire digestive tract, in the stomach where the pH is too low for the activity of most phytases, and maintaining its activity in the small intestine where the pH is close to 7.

In the attached figures:
Figure 1 shows the determination of the molecular weight of the protein with phytase activity.
Figure 2 shows the determination of the enzymatic activity of the protein with phytase activity according to the temperature.
Figure 3 shows the determination of the enzymatic activity of the protein with phytase activity according to the pH.
Figure 4 shows the effect of proteases on the enzymatic activity.
Figure 5 shows the maintenance of the phytase activity over time.

Therefore, according to one aspect of the invention, an isolated *Serratia odorifera* gene encoding a protein or a polypeptide with phytase activity is provided. The protein or the polypeptide encoded by said *Serratia odorifera* gene with phytase activity expressed in a yeast host strain, preferably *Pichia pastoris,* wherein the protein or the polypeptide has allosteric properties which double the enzymatic activity thereof (measured according to ISO30024:2009 (E)) four hours after coming into contact with the substrate and remaining active for more than eight hours, is also an object of the invention.

Thus, the present invention provides a gene encoding a protein or a polypeptide with phytase activity, its nucleotide sequence being the one shown in SEQ ID No 2, or a derivative thereof.

In another aspect, the invention provides a protein or a polypeptide with phytase activity with the following characteristics: a) Theoretical molecular weight of 46 kDa; b) optimal pH range of 3.5 to 5.8; c) temperature of optimal activity in the range of 45-55°C; d) High specific activity of 1120 ± 150 U/mg, particularly of 1,123 ± 112 U/mg; e) high resistance to pepsin and trypsin, and f) isoelectric point PI=6.1, wherein the protein with phytase activity is selected from among:
i) a polypeptide with phytase activity comprising the amino acid sequence shown in SEQ ID No 3 or a derivative thereof by substitution, deletion or insertion of one or more amino acid residues, or.
ii) a polypeptide with phytase activity which is encoded by a polynucleotide of SEQ ID No 2 or a polypeptide with phytase activity having a sequence homology of at least 60% with SEQ ID No 2, preferably at least 65%, 70%, 75%, 80%, 85%, 90% or 95%, with special preference at least 95%.

This phytase is obtained from a microorganism of the *Serratia* genus, preferably from *Serratia odorifera.*

Likewise, according to another aspect of the invention, the use of the protein encoded by said *Serratia odorifera* gene with phytase activity expressed in a yeast strain as a host, as an active ingredient, in the manufacture of additives for feeds intended for animal nutrition, is another object of the invention.

According to the first aspect of the invention, an isolated *Serratia odorifera* gene according to SEQ ID No 2 is provided, which gene encodes a protein or a polypeptide with phytase activity, the protein having the following characteristics:
a) Theoretical molecular weight of 47 kDa;
b) Optimal pH range of 3.5 to 5.8; preferably from 3.7 to 5.8;
c) Temperature of optimal activity in the range of 45-55°C, preferably at 50°C;
d) High specific activity of 1120 ± 150 U/mg;
e) High resistance to pepsin and trypsin; and
f) Isoelectric point of 6.1.

The protein or the polypeptide of the invention encoded by said *Serratia odorifera* gene with phytase activity is expressed in a yeast host strain, preferably *Pichia pastoris.* Thus, in another aspect of the invention, it relates to a recombinant vector comprising the *Serratia odorifera* gene encoding the protein or the polypeptide with phytase activity, a recombinant host cell, such as *Pichia pastoris,* in which said vector has been introduced, as well as a method for expressing the enzyme in a host cell.

In the present context, "polypeptide with phytase activity comprising the amino acid sequence shown in SEQ ID No 3 or a derivative thereof by substitution, deletion or insertion of one or more amino acid residues" is understood, for example in the case of substitutions, as conservative amino acid substitutions well known by the person skilled in the art and which do not modify the phytase activity, being likewise applicable to deletions or insertions. It is general knowledge for the person skilled in the art that, during gene cloning, recognition sites are designated which could result in one or more unrelated amino acid residues at the ends of the target protein, without the phytase activity therefore being affected. It is likewise known that for the purpose of constructing a fusion protein, improving the expression of a recombinant protein, obtaining a recombinant protein secreted by the host cell or facilitating the purification of the recombinant protein, in the N-terminus or in the C-terminus, or in other suitable regions of the proteins, binding peptides, signal peptides, leader peptides, terminal extensions or the like are usually introduced.

The invention likewise provides a polypeptide with phytase activity which is encoded by a polynucleotide of SEQ ID No 2 or a polypeptide with phytase activity having a sequence homology of at least 60% with SEQ ID No 2, preferably at least 65%, 70%, 75%, 80%, 85%, 90% or 95%, with special preference at least 95%. In this respect, it will be understood that the invention furthermore includes nucleic acid molecules which differ in any of the nucleotides shown in SEQ ID No 2 due to the degeneration of the genetic code, provided that they encode the same phytase protein.

In the present invention, the term "vector" refers to a nucleic acid which is capable of transporting another nucleic acid to which it is bound, being able to be, for example, a plasmid or a viral vector.

Thus, the recombinant vectors of the invention comprise a nucleic acid of the invention in a suitable form for the expression in a host cell, which means that the recombinant vectors of the invention include one or more regulatory sequences, selected according to the host cell to be used for the expression, operatively bound to the sequence to be expressed; i.e., the nucleotide sequence of interest binds to the regulatory sequence such that it allows the expression of the nucleotide sequence. In this respect, it is understood that the regulatory sequence can include promoters, activation sites, terminators, etc. known by the person skilled in the art.

The vector can be introduced in the host cell by means of conventional transformation or transfection techniques. Methods of this type for transforming or transfecting host cells can be found, for example, in Sambrook et al., Molecular cloning: A laboratory Manual, 2nd Ed., Cold Spring Harbor Laboratory Press, 1989.

In the present invention, the host cell is used to produce a protein with phytase activity. Therefore, the invention provides a method for producing a protein with phytase activity as has been described above using a yeast host cell, preferably of *Pichia pastoris,* which comprises the *Serratia odorifera* gene, which encodes a protein with phytase activity, wherein the method includes culturing the host cell in which the expression vector encoding the protein with phytase activity has been introduced, in a medium suitable for the production of the protein and subsequent separation of the proteins from the medium, if they are secreted, or from the host cell.

According to another aspect of the invention, another object thereof is the use of the protein encoded by said *Serratia odorifera* gene with phytase activity expressed in a yeast strain as a host, as an active ingredient, in an effective amount, in the manufacture of additives for feeds intended for animal nutrition, and also the additive thus obtained.

The additive of the invention can be suitably prepared in solid or liquid form and can optionally include other enzyme preparations correspondingly suitable for the solid or liquid preparation, being suitable for use in the preparation of feeds or other foods intended for animal nutrition since it contains an effective amount of phytase which favors the assimilation of phosphorus and increases the absorption of metals and proteins upon releasing them from phytate.

### Examples

### Example 1: Expression in Pichia pastoris

### Construction of the expression vector

To isolate the region encoding the mature protein, the following oligonucleotides were synthesized:
SerrF: GCGCGCGAATTCGAGCCGCGCTACGTATTGG
SerrR: GCGCGCAAGCTTGTCTAGACGTGGCCGGTACTGCACCG

The region encoding the mature protein was amplified using the oligonucleotides serrF and SerrR. The amplification product was visualized in an agarose gel, the band of the expected size was cut and the DNA was extracted from the gel by means of the NucleoSpin Extract II kit (Macherey-Nagel).

The purified DNA was inserted inside the vector pPICZα A (Invitrogen, San Diego, Calif.) between the restriction sites delimited by the EcoRI and XbaI sequences. The construct was transformed into DH5α cells which were subsequently plated in LB medium containing 25 µg/ml Zeocin. The positive colonies were sequenced. One of the colonies with the correct sequence was cultured in large amounts for the purpose of obtaining DNA for transforming the yeasts.

### Transformation of the yeasts and expression of the protein

The *Pichia pastoris* strain KH71H (Invitrogen) was grown in YPD medium and prepared for the transformation. 10 µg of plasmid pPICZα A AppAs-r was linearized by means of the restriction enzyme PmeI and introduced in the yeasts by means of shock with Lithium/Polyethylene glycol.

The transformed cells were plated in YPDZ medium (it contains 100 µg/ml Zeocin) which allows selecting the colonies which have integrated the Sh ble gene (the product of this gene confers resistance to Zeocin) in the chromosomal DNA of the host. The transformants which have integrated the Sh ble gene will grow correctly in the YPDZ medium. After 2 days, the transformants were inoculated in minimum medium with glycerol (BMGY) for 24 hours, the yeasts were then recovered by means of centrifugation (2,500g, 3 minutes) and resuspended in a medium with 0.5% methanol (BMMY) to induce the expression of the phytase gene.

### Quantification of the phytase activity of the transformants

A total of 61 transformants were analyzed to quantify the phytase activity by means of the molybdate-vanadate method in acidic medium (established in ISO 300024:2009 (E)).

1,160 µl of substrate solution (5 mM sodium phytate in a 250 mM sodium acetate buffer, pH 5.5) were added to 40 µl of a diluted solution of the enzyme. The reaction was carried out at 37°C for 30 minutes. 800 µl of STOP solution (14% nitric acid; 3.3% ammonium heptamolybdate tetrahydrate, 0.078% vanadate) were then added to the reaction to stop the reaction. As a control, the STOP solution was added to the diluted solution of the enzyme to inactivate it and then the substrate solution was added. After 10 minutes, the yellow color intensity at 415 nm was quantified and the activity in solution of the enzyme and of the control solution were calculated. One unit of phytase activity is defined as the amount of enzyme releasing 1 µmol of phosphorus in one minute. Two days after the induction by methanol, 6 transformants of 61 produced between 10 and 55 U/ml of culture medium. The gene obtained is obviously a novel gene encoding a polypeptide with phytase activity. The polypeptide with phytase activity was referred to as AppAs-r.

### Example 2: Purification of AppAs-r expressed in Pichia pastoris

For the purpose of purifying the phytase produced by *Pichia pastoris,* the transformant with an activity of 55 U/ml was cultured and induced in optimal conditions. Four days after the induction with methanol, the phytase activity was measured by means of the same method used in the previous example, resulting in an activity of 163 U/ml in the supernatant. The supernatant was obtained by means of centrifugation at 14,100 g for 5 minutes. The cell precipitate was discarded.

The supernatant was concentrated by means of centrifugation with VIVASPIN 20 (50,000 MWC O (Sartorius Studium)). Next, the concentrated solution was dialyzed in sodium acetate buffer (0.25 M, pH 5.5). Finally, the phytase was purified by means of gel filtration (Sephacryl S-200) with the same buffer used in the dialysis.

### Example 3: Characteristics of AppAs-r expressed in Pichia pastoris

### Determination of the molecular weight

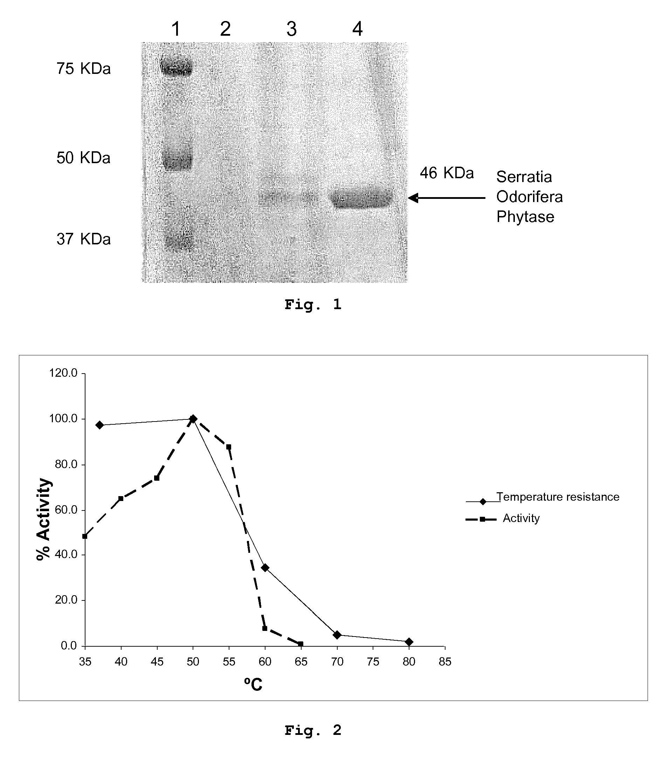

The molecular weight of phytase AppAs-r was characterized by means of an acrylamide denaturing gel (SDS-PAGE). In Figure 1, line 1 corresponds to the molecular weight marker; line 2 refers to the control of culture medium without induction with methanol; line 3 to the culture medium induced with methanol; and line 4 to the dialyzed and purified concentrated phytase.

As shown in Figure 1, the molecular weight of AppAs-r is approximately 46 kDa, which is the weight estimated from the polypeptide sequence derived from the cloned DNA sequence.

### Determination of the enzymatic activity of AppAs-r with temperature

As observed in Figure 2, the activity changes with the temperature. The optimal temperature was determined at a pH of 5.5, since this is the pH demanded by ISO 300024:2009 (E). A temperature range between 35°C and 80°C was tested. The maximum activity was registered at 50°C. As shown in Figure 2, after 10 minutes at 60°C the enzyme retains 40% of its activity.

### Determination of the enzymatic activity of AppAs-r dependent on pH

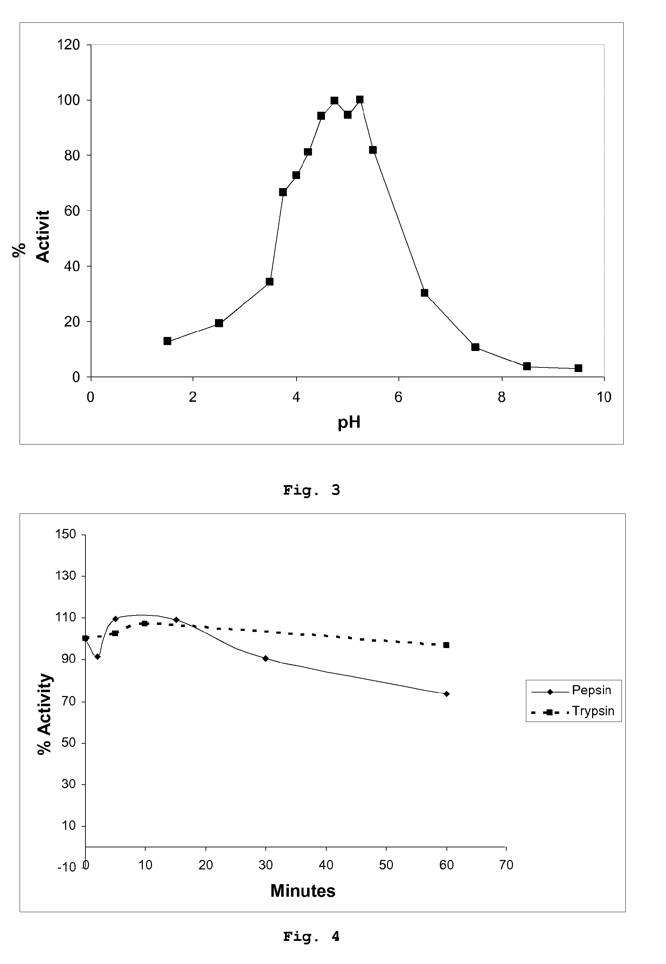

The effect of pH on phytase activity was checked using the following buffers: Glycine-HCl for pH 1.5-3.5; sodium acetate for pH 3.5-6.0; Tris-HCl for pH 6.0-8.5; Glycine-NaOH for pH 8.5-10.0. All the buffers contained 0.05% IGPAL. As observed in Figure 3, the enzymatic activity varies with the pH. The maximum activity was observed at a pH 4.75. 70% of the activity is maintained at a pH of 3.75, whereas more than 80% of the activity is maintained at a pH of 5.5.

### Effect of proteases on the enzymatic activity

To determine the resistance to proteases, purified AppAs-r (1.6 mg/ml) was incubated with 5 mg/ml pepsin or with 50 mg/ml trypsin at 37°C. After the incubation at different times (3; 5; 10; 15; 30 and 60 minutes), the phytase activity remaining in the samples was quantified. As can be observed in Figure 4, after 1 hour of incubation with pepsin, phytase AppAs-r retains more than 70% of its initial activity, whereas it retains more than 90% of its initial activity after 1 hour of incubation with trypsin. Thus, this phytase is highly resistant to the action of proteases, a fact which is very beneficial for its use in animal food.

### Maintenance of the phytase activity with time

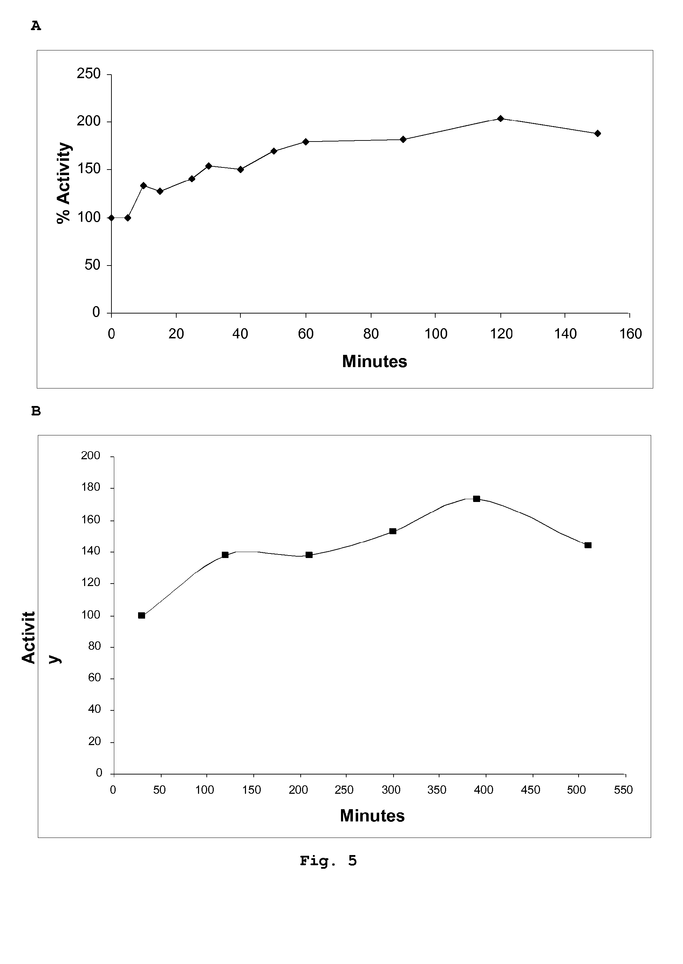

To determine the activity kinetics over time of phytase AppAs-r, the specific activity per minute was measured at different times after incorporating the substrate. As observed in Figure 5A, unlike most phytases, the specific activity of AppAs-r increases over time. As shown in Figure 5B, AppAs-r is active for at least 8 hours from the time at which the substrate is added, doubling its initial specific activity after 4 and a half hours of activity. This increasing activity sustained over time has not been described in any other phytase and is a very important point since animal digestion lasts more than 30 minutes and it is important for the enzyme to be active for as long as possible. This increasing activity sustained over time can also be very relevant in liquid food. This would allow releasing most of the phosphorus present as phytate in the feeds with a much smaller amount of enzyme.

### Determination of the specific activity

For the purpose of determining the specific activity, the concentration of the concentrated and purified phytase AppAs-r was assessed by means of the Lowry method and the specific activity was studied by means of the molybdate-vanadate acidic method at 37°C and a pH of 4.75. As a result, the specific activity of purified AppAs-r was 1,123 ± 112 U/mg.

### Example 4: Release of phosphate from organic matter coming from soil

Due to the long active life of phytase and with the intention of knowing its capacity to release inorganic phosphorus from organic matter coming from soil, the purpose of this assay being to study its use in the preparation of an additive for fertilizers, 5 samples of soil were collected. 2.5 g of each one were weighed and resuspended in an acetate buffer, calculating a final concentration of 0.25 g of sample/ml. Incubation for 20 minutes with stirring at room temperature was performed. A 1/10 dilution of the samples in the buffer acetate was then carried out. The equivalent to 1 U of phytase AppAs-r/100g soil was added to 1.2 ml of each diluted sample and it was incubated for 24 hours at 25°C. As a result, the release of phosphorus was 53.6 ± 27.8 mg of phosphorus/100 g of soil, increasing the free phosphate of the soil by 23.4 ± 18.1%.

## Claims

1. A protein with phytase activity with the following characteristics:
a) Theoretical molecular weight of 46 kDa;
b) Optimal pH range of 3.5 to 5.8;
c) Temperature of optimal activity in the range of 45-55°C;
d) High specific activity of 1120 ± 150 U/mg;
e) High resistance to pepsin and trypsin; and
f) Isoelectric point of 6.1.

2. The protein with phytase activity according to claim 1, **characterized in that** the optimal pH range is from 3.7 to 5.8, the optimal temperature is 50°C and the specific activity is 1,123 ± 112 U/mg.

3. The protein with phytase activity according to claims 1-2, **characterized in that** it is selected from among:
i) a polypeptide with phytase activity comprising the amino acid sequence shown in SEQ ID No 3 or a derivative thereof by substitution, deletion or insertion of one or more amino acid residues, or
ii) a polypeptide with phytase activity which is encoded by a polynucleotide of SEQ ID No 2 or a polypeptide with phytase activity having a sequence homology of at least 60% with SEQ ID No 2, preferably at least 65%, 70%, 75%, 80%, 85%, 90% or 95%, with special preference at least 95%.

4. The protein according to claim 1, **characterized in that** it is encoded by a *Serratia odorifera* gene of SEQ ID No 2 and expressed in a yeast strain.

5. The protein according to claim 4, **characterized in that** the yeast strain is *Pichia pastoris.*

6. A recombinant vector comprising the *Serratia odorifera* gene encoding the protein with phytase activity according to claim 1 and a *Pichia pastoris* recombinant host cell in which said vector has been introduced.

7. A process for producing a protein with phytase activity which comprises culturing the *Pichia pastoris* host cell in which the vector according to claim 6 has been introduced.

8. Use of the protein according to claim 1 encoded by the *Serratia odorifera* gene with phytase activity expressed in a yeast strain as a host, as an active ingredient, in a effective amount, in the manufacture of additives for feeds intended for animal nutrition.

9. An additive for animal food, including an effective amount of a protein according to claim 1.

10. The additive according to claim 9, **characterized in that** it is suitably prepared in solid or liquid form and **in that** it can include other enzyme preparations correspondingly suitable for the solid or liquid form.

11. An additive for fertilizers, including an effective amount of a protein according to claim 1.


SEQUENCE LISTING

<110> xxxxx, xxxxx

<120> Novel phytase, process for obtaining and using same

<160> 2

<170> BiSSAP 1.0

<210> 1
<211> 1281
<212> DNA
<213> Serratia odorifera

<220>
<221> source
<222> 1..1281
<223> /mol_type="DNA"
/organism="Serratia odorifera"

<400> 1
atgttgctat tgcaaaagga ctggtcgcgt ctgttatttg ccgtcacgct gggtatgatt 60
tccagcgtag cccaggctga gccgcgctac gtattggaaa aggtggttga ggtcagccgc 120
cacggcgtac gcccgccgac ctcaggcaac cggcaggcga tgcaggcggg aaccggccga 180
gagtggccac aatggctgac gcgcgacggc gaactcactg gccacggtta tgccgccgcc 240
acgctgaaag gacgctatga agccgactat tatcgccgtc agggcctatt ggccaacggc 300
tgtccgagcg cgggggcggt gtatgtctgg gccagtccgc tacagcgcac gcgagccacc 360
gcacaggcgt tgatggacgg cgcatttccc ggctgcgggg tcgccattca tgcggccgcc 420
accgaacagg accccctgtt tcaggcagat aaaatgggcc tggtgccgct cgatgccgaa 480
cgggctcgca cggcaataag gcaggcaatg ggcggcagcg ccgagcaggt gaaaacgcgc 540
tttagcgctg acattcggcg tctgcaagcg gcggtctgcc tgccgcaaca ggcttgcccg 600
gcctttgaac aaccgtggga aatcactcag gagcacgacg gccgcttcag catcaacggt 660
ctggggacgt tgtccaacat ggcggaaagc attcgcctgg cctacagcga aaaccagccg 720

acggcgcagg tcgcctttgg ccacggtgtc aacgcatcgg ccgtcgcgcc gttgctgccc 780

ctgctcaccg cccgctatga ctttaccaat gacgtgccct atatcgcgca acgcggtggc 840

tcggtgctgt taaaccaaat cgcgctggcg ctggccgccg atcgaacctc tgccggggcg 900

ccaccggcgg cgcgctggtt gctgtttgtc gcgcatgaca ccaatatcgc ttatctgcgc 960

accctgcttg gctttagctg gcaacagggg ctttacccac gcggcaatat tcccccggct 1020

ggcagtctgg tattcgaacg ctggcgcgat cggcaaacgg gccagcgctt cctgcgtctg 1080

tacttccagg cgcaatcgct ggatcaaatc cgccagttgt caccgctgag cacgctgtcg 1140

ccaccgttaa aaaccgagtt cagccgtcct ggctgccggc agttgtcact gggcgtactc 1200

tgtccctgga ctgagtcgat gcaacggatg cgcgcggcta tcgacccgac ggcgctgcct 1260

acggtgcagt accggccata a 1281

<210> 2
<211> 426
<212> PRT
<213> Pichia pastoris

<220>
<221> SOURCE
<222> 1..426
<223> /mol_type="protein" /organism="Pichia pastoris"
<400> 2

Met Leu Leu Leu Gln Lys Asp Trp Ser Arg Leu Leu Phe Ala Val Thr
1               5                   10                  15
Leu Gly Met Ile Ser Ser Val Ala Gln Ala Glu Pro Arg Tyr Val Leu
            20                  25                  30
Glu Lys Val Val Glu Val Ser Arg His Gly Val Arg Pro Pro Thr Ser
        35                  40                  45
Gly Asn Arg Gln Ala Met Gln Ala Gly Thr Gly Arg Glu Trp Pro Gln
    50                  55                  60
Trp Leu Thr Arg Asp Gly Glu Leu Thr Gly His Gly Tyr Ala Ala Ala
65                  70                  75                  80
Thr Leu Lys Gly Arg Tyr Glu Ala Asp Tyr Tyr Arg Arg Gln Gly Leu

85                  90                  95
Leu Ala Asn Gly Cys Pro Ser Ala Gly Ala Val Tyr Val Trp Ala Ser
            100                 105                 110
Pro Leu Gln Arg Thr Arg Ala Thr Ala Gln Ala Leu Met Asp Gly Ala
        115                 120                 125
Phe Pro Gly Cys Gly Val Ala Ile His Ala Ala Ala Thr Glu Gln Asp
    130                 135                 140
Pro Leu Phe Gln Ala Asp Lys Met Gly Leu Val Pro Leu Asp Ala Glu
145                 150                 155                 160
Arg Ala Arg Thr Ala Ile Arg Gln Ala Met Gly Gly Ser Ala Glu Gln
                165                 170                 175
Val Lys Thr Arg Phe Ser Ala Asp Ile Arg Arg Leu Gln Ala Ala Val
            180                 185                 190
Cys Leu Pro Gln Gln Ala Cys Pro Ala Phe Glu Gln Pro Trp Glu Ile
        195                 200                 205
Thr Gln Glu His Asp Gly Arg Phe Ser Ile Asn Gly Leu Gly Thr Leu
    210                 215                 220
Ser Asn Met Ala Glu Ser Ile Arg Leu Ala Tyr Ser Glu Asn Gln Pro
225                 230                 235                 240
Thr Ala Gln Val Ala Phe Gly His Gly Val Asn Ala Ser Ala Val Ala
                245                 250                 255
Pro Leu Leu Pro Leu Leu Thr Ala Arg Tyr Asp Phe Thr Asn Asp Val
            260                 265                 270
Pro Tyr Ile Ala Gln Arg Gly Gly Ser Val Leu Leu Asn Gln Ile Ala
        275                 280                 285
Leu Ala Leu Ala Ala Asp Arg Thr Ser Ala Gly Ala Pro Pro Ala Ala
    290                 295                 300
Arg Trp Leu Leu Phe Val Ala His Asp Thr Asn Ile Ala Tyr Leu Arg
305                 310                 315                 320
Thr Leu Leu Gly Phe Ser Trp Gln Gln Gly Leu Tyr Pro Arg Gly Asn
                325                 330                 335
Ile Pro Pro Ala Gly Ser Leu Val Phe Glu Arg Trp Arg Asp Arg Gln
            340                 345                 350
Thr Gly Gln Arg Phe Leu Arg Leu Tyr Phe Gln Ala Gln Ser Leu Asp
        355                 360                 365
Gln Ile Arg Gln Leu Ser Pro Leu Ser Thr Leu Ser Pro Pro Leu Lys
    370                 375                 380

Thr Glu Phe Ser Arg Pro Gly Cys Arg Gln Leu Ser Leu Gly Val Leu
385                 390                 395                 400
Cys Pro Trp Thr Glu Ser Met Gln Arg Met Arg Ala Ala Ile Asp Pro
                405                 410                 415
Thr Ala Leu Pro Thr Val Gln Tyr Arg Pro
            420                 425